Europäisches Patentamt

European Patent Office

Office européen des brevets

(11) Publication number: **0 416 793 A1**

# EUROPEAN PATENT APPLICATION

(21) Application number: **90309367.2**

(22) Date of filing: **28.08.90**

(51) Int. Cl.5: **A61B 17/22, A61N 1/05**

(30) Priority: **30.08.89 US 400701**
**30.08.89 US 400702**

(43) Date of publication of application:
**13.03.91 Bulletin 91/11**

(84) Designated Contracting States:
**CH DE DK FR GB IT LI NL**

(71) Applicant: **ANGEION CORPORATION**
**13000 Highway 55**
**Plymouth, Minnesota 55441(US)**

(72) Inventor: **Isner, Jeffrey M.**
**736 Cambridge Street**
**Boston MA 02135(US)**
Inventor: **Clarke, Richard**
**590 Memorial Drive**
**Boston MA 02135(US)**

(74) Representative: **Parr, Ronald Edward R.E. Parr**
**& Co.**
**Colman House Station Road**
**Knowle Solihull West Midlands B93 0HL(GB)**

(54) Catheter.

(57) Apparatus for use in the treatment of diseases of the human heart comprises:
  a. a medical laser;
  b. an optical fiber (52) operatively connected to the medical laser;
  c. advancing means (30, 42) for percutaneously advancing the optical fiber to a position adjacent a human heart; and
  d. means for locating and/or maintaining the optical fiber in said position adjacent a human heart.

FIG. 1

# BACKGROUND OF THE INVENTION

## 1. Field of the Invention

The invention relates to apparatus useful in treatment of heart diseases, for example ventricular tachycardia, hypertrophic cardiomyopathy (HCM) and idiopathic hypertrophic subaortic stenosis (IHSS), and more particularly, in percutaneous treatment using laser energy (ablation).

## 2. Description of the Prior Art

### (a) IHSS and HCM

In these diseases an enlargement of the tissue of the chamber wall within the heart interferes with normal cardiac function. In addition to the standard treatments for impaired cardiac function, the problem has been addressed surgically. Andrew G. Morrow, M.D., et al. discusses such a surgical approach in "Operative Treatment in Idiopathic Hypertropic Subaortic Stenosis", Circulation Volume XXXVII, April 1968, pages 589-596. Though the clinical results of Morrow appear to be promising, they indicate that care must be exercised because an effective operation requires that the knife must be "plunged into the septum until it is out of sight, completely."

The problem of performing the cardiomyoplasty is in part resolved by Jeffrey M.Isner, M.D., et al ., in "Laser Myoplasty for Hypertrophic Cardiomyopathy", American Journal of Cardiology, Volume 53, 1984, pages 1620-1625. Isner teaches the accomplishment of the procedure by the technique of photoablation using an argon laser. However, the main difficulty with the techniques of Morrow and of Isner is the requirement to perform a thoractomy. The difficulty of performing a thoractomy and the added mortality is well known.

### (b) Ventricular tachycardia

This is a disease of the heart in which the heart's normal rhythmic contraction is altered, thus affecting left ventricular function. The condition is often described as a heart beat which is too fast, although the disease is far more complex. In many patients the orderly contractions of the heart are not present because various portions of the myocardium do not contract and relax in proper synchronism with the rest of the heart.

Currently, the most common treatment for tachycardia is through the use of various drugs. Some drugs reduce the irritability of the offending myocardial tissue, whereas other drugs may slow the response time of all myocardial tissue. In either case the treatment is administered systemically resulting in various side effects.

A second technique now gaining some popularity is surgical intervention. Initially, surgery was performed only in those circumstances which were totally refractory to drug therapy; however, surgical intervention is now more popular. The technique involves electrophysiological mapping of the myocardium. Foci of the tachycardia are located and surgically excised. The major disadvantage of this procedure is the cost and risk associated with open chest cardiac surgery. A description of the surgical procedure may be found in "Endocardial Excision: A new Surgical Technique for the Treatment of Recurrent Ventricular Tachycardia", by Mark E. Josephson, M.D., et al , Circulation, Volume 60, Number 7, 1979.

A first variation on surgical excision involves cryosurgery. In this technique, the diseased myocardial tissue is destroyed by freezing. See for example "The Successful Cryosurgical Treatment of Paroxyal Ventrical Tachycardia", Chest, Volume 75, at page 612, 1979. Other variations involve the use of DC shock and radiofrequency energy.

Perhaps the most promising technique similar to surgical excision is with the use of laser energy. Such a technique is described in "Neodymium: YAG Laser Photocoagulation: A Successful New Map-Guided Technique for the Intraoperative Ablation of Ventricular Tachycardia", by Robert H. Svenson, M.D., et al ., Circulation, Volume 76, Number 6, December, 1987. This technique attempts to photocoagulate rather than ablate the diseased tissue. In this way the mechanical integrity of the myocardium is preserved. However, because the method of Svenson is intraoperative, the major problem with surgical excision (i.e. need to perform a thorocotomy) is not overcome by this technique. The difficulty that remains is the cost and trauma associated with this procedure.

# SUMMARY OF THE INVENTION

The present invention overcomes the difficulties in the prior art treatments by use of novel apparatus including a novel catheter to photoablate that tissue which impairs cardiac function. The most important advantage is that the use of a percutaneous procedure does not require thoractomy thus significantly reducing cost, time, trauma and mortality rate.

Thus, according to a first aspect the present invention provides apparatus for treating diseases of the human heart, characterised in that it comprises:

a. a medical laser;

b. an optical fiber (52) operatively connected to the medical laser;

c. advancing means (30,42) for percutaneously advancing the optical fiber to a position adjacent a human heart; and

d. means for locating and/or maintaining the optical fiber in said position adjacent a human heart.

According to a second aspect the present invention provides a catheter assembly for use in the apparatus of the first aspect of the invention, the catheter assembly comprising:

a first, outer flexible sheath (18);

a second, inner flexible sheath (22) removably disposed within the outer sheath and suitable to receive an optical fiber (52) and also an elongate locating and/or maintaining means (42) to locate and/or maintain a distal end of the optical fiber in or adjacent the heart;

a branched tubular housing (26) having a first tubular member (28) the distal end of which can engage the proximal end of the outer sheath (18), and a second tubular member (32) extending from the first tubular member at a position intermediate the proximal and distal ends of the first tubular member whereby the interior of the first and second tubular members are in communication with each other, the first tubular member being suitable to receive therethrough the optical fibre (52) and the second tubular member being suitable to receive therethrough the elongate means (42).

Preferred methods of using the apparatus and the catheter assembly of the invention are described below, first with reference to HCM and IHSS and, secondly, with reference to ventricular tachycardia.

(a) Re: HCM and IHSS

The apparatus and catheter assembly of the present invention can be used in a new method to treat IHSS or HCM using a percutaneous approach obviating the need for the interoperative Morrow procedure. In a preferred form, the method is to percutaneously deliver a catheter via the femoral artery or vein to the septal wall of the left ventrical. The catheter is fixed to the wall by a fixation device after which the laser fiber optic tube is inserted into the catheter and positioned at the distal end. The fiber optical assembly is secured to a laser which is activated to irradiate the tissue. After sufficient volume reduction is achieved by repeated use of laser energy, the device is removed.

In a typical percutaneous procedure to treat IHSS a preferred catheter assembly consisting of a fiber optic tube, an automatic fixation device, a delivery catheter and a guiding catheter with associated connectors is inserted into the human body either in a retrograde fashion through the femoral artery or transceptually through the femoral vein. The catheter is affixed to the septal wall in the hypertrophied region by means of a fixation device contained within the catheter. The laser is then energized for a period of time photocoagulating or ablating the irradiated myocardial tissue. The thermal damage caused by photocoagulation creates a local myocardial infarction with subsequent reduction in tissue volume. This change decreases the thickness of the septal wall reducing the outflow track gradient and restoring more normal left ventricular performance.

In photocoagulation, part of the laser energy is absorbed by the tissue directly underneath the fiber optic probe and part is scattered throughout the tissue, eventually being totally absorbed over a much greater area than the diameter of the fiber optic tube. The absorbed energy raises the temperature of the tissue resulting in a controlled injury and reduced volume of the affected tissue.

(b) Re: Ventricular tachycardia

The present invention alleviates the cost and trauma associated with a thorocotomy. In a preferred procedure, after the myocardium has been mapped and the sites to be treated have been indentified, the catheter assembly of the invention is introduced percutaneously and advanced into the left ventricle either transarterially in retrograde fashion, or transceptually after transveneous introduction. The distal tip is positioned adjacent a site to be treated and maintained in position with fixation means. Energy from a medical laser is supplied to the site via an optical fiber within the special catheter. Upon being irradiated, the myocardial tissue at the identified site is photocoagulated or photoablated. Other sites identified during the mapping process are similarly treated. Because the technique is performed percutaneously without bypass, each ablated or coagulated focus may be immediately tested to ensure that the corresponding tachycardia mode has been successfully treated.

BRIEF DESCRIPTION OF THE DRAWINGS

There are now described, by way of example and with reference to the accompanying drawings, apparatus and a catheter assembly according to preferred embodiments of the first and second aspects, respectively, of the invention.

In the drawings:

FIG. 1 is a perspective (plan) view of the catheter assembly of the invention;

FIG. 2 is a cutaway view of a piston and cylinder assembly (also referred to as a "syringe") and the proximal end of a guide (fixation) wire;

FIG. 3 is a cutaway view of the proximal end of the catheter showing entry of the optical fiber;

FIG. 4 is a cutaway view of the proximal end of the outer sheath;

FIG. 5 is a cutaway view of the distal end;

FIG. 6 is a schematic diagram of procedure using a retrograde femoral approach;

FIG. 7 is a cutaway view of the heart during ablation using a retrograde femoral approach for treatment of HCM or IHSS;

FIG. 7A is a cutaway view of the heart using a retrograde femoral approach for treatment of ventricular tachycardia;

FIG. 8 is a schematic diagram of the procedure using a transceptual approach;

FIG. 9 is a cutaway view of the heart during ablation using a transceptual approach for treatment of HCM or IHSS; and

FIG. 9A is a cutaway view of the heart during ablation using a transceptual approach for treatment of ventricular tachycardia.

## DESCRIPTION OF THE PREFERRED EMBODIMENTS

The present invention concerns, inter alia a technique for the percutaneous treatment of idiopathic hypertrophic subaortic stenosis (IHSS) and hypertrophic cardiomyopathy (HCM). In IHSS the septal wall near the aortic valve thickens reducing the performance of the left ventricle by partially or completely occluding the orifice. In HCM the thickness of the myocardium increases to the extent that the chamber size is reduced, thereby limiting stroke volume.

The common treatment for either disease is surgically to reduce the thickness by removing some of the muscle tissue (i.e., performing a myectomy) or reforming the myocardium to improve the shape of the inside of the chamber and increase its volume (i.e., cardiomyoplasty). The reforming can be done surgically (i.e., myoptomy) or by inducing a controlled infarct. The present invention provides apparatus and catheter assembly for performing these procedures percutaneously using laser energy.

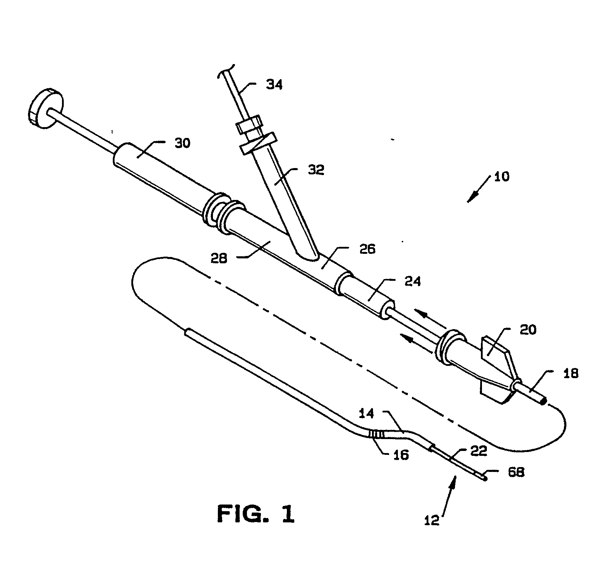

FIG. 1 is a plan view of catheter (10) of the subject invention. One purpose of catheter (10) is to transmit energy from a medical laser to the myocardium. This transfer may be transarterial or transveneous as described below.

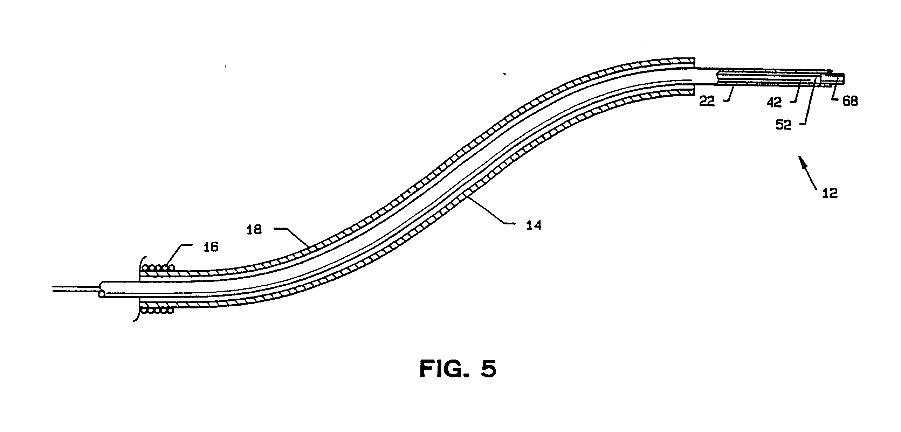

Laser energy is directed to the tissue from distal tip (12). A more detailed view of distal tip (12) is found in FIG. 5. Distal tip (12) is held in position within the ventricle by preformed sigmoidal bend (14) of guiding sheath 18 and fixation wire (42). Distal metal ring (16) provides a radiopaque indication of the location of distal tip (12). For ease of grasping and turning guiding sheath (18), it contains winged member (20) at its proximal end. The distal end of a Y-shaped housing (26) (referred to also as "wye 26") frictionally engages the proximal end of guiding sheath (18 ) during use, but is shown exploded in FIG. 1 to reveal the detail.

Inner catheter (22) runs the entire length of guiding sheath (18). Inner catheter (22) contains the inner lumen through which runs the optical fiber (52) for transmission of the laser energy and the fixation wire (42). Inner catheter (22) is frictionally coupled via swagging or thermoplasty to metal tubing (24) which runs most of of the length of wye (26) and defines the inner lumen of main branch (28) of wye (26). A cylinder and mating piston assembly (30) (referred to also as "syringe 30") frictionally engages main branch (28) of wye (26).

Secondary branch (32) of wye (26) receives sheath (34) which contains the optical fiber through which the laser energy is transmitted.

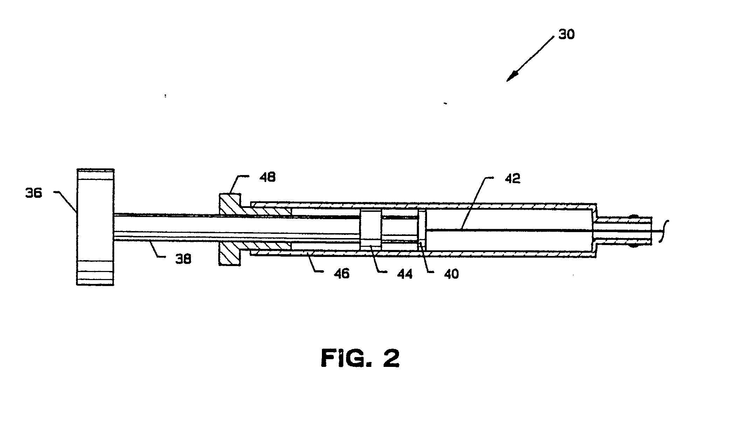

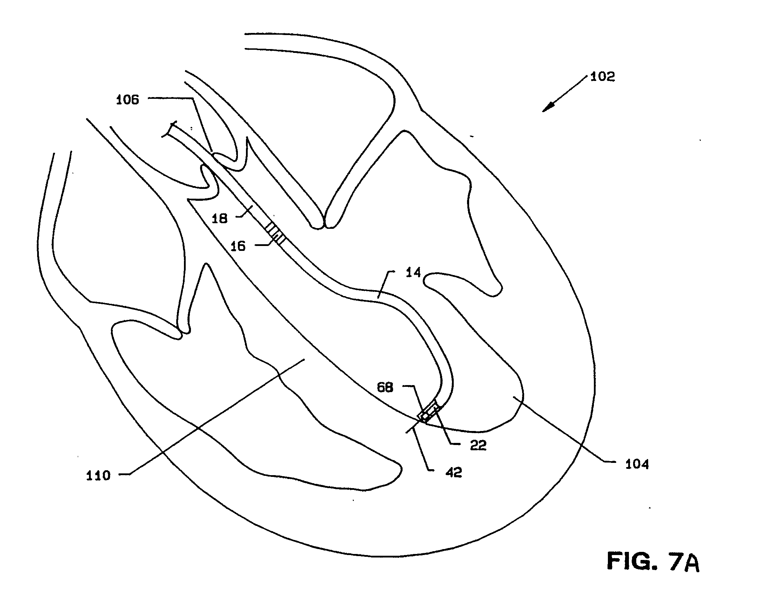

FIG. 2 is a cutaway view of syringe 30. At its most proximal end is thumb knob 36. Depressing thumb knob 36 moves shaft 38 distally which moves piston 40 distally. Fixation wire 42, which runs the entire length of catheter 10, is fixedly attached to piston 40 and is therefore moved distally by pressing thumb knob 36. Fixation wire 42 is substantially stiffer than the inner catheter 22 of catheter 10. The movement of thumb knob 36 (and hence fixation wire 42) in the distal or proximal direction permits medical personnel to fix the position of distal tip 12 of catheter 10 (see also FIG. 1) and to penetrate the heart tissue for stability (see also FIG. 7).

Rubber seal 44 sealingly engages wall 46 of syringe 30. Configured stopper 48 guides the movement of shaft 38 for smooth operation. Because syringe 30 is airtight, it may be used for resisting inadvertent proximal or distal movement of fixation wire 42.

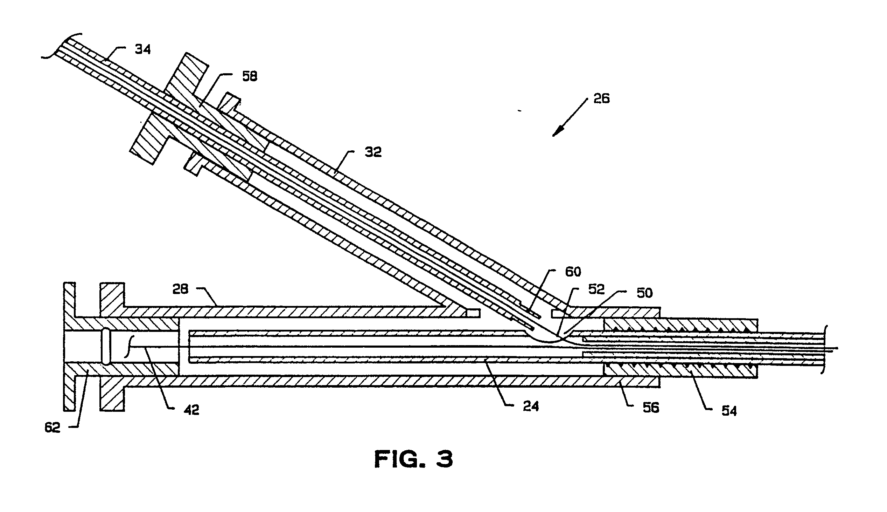

FIG. 3 is a cutaway view of wye 26. The outer structure is a molded, rigid plastic. It has a main branch 28 into which syringe 30 is inserted and a secondary branch which receives the optical fiber. As explained above the main branch contains metal tubing 24 which provides a lumen for fixation wire 42. Metal tubing 24 has an aperture 50 which is Positioned to receive optical fiber 52. Metal tubing 24 is fixedly engaged by rigid plastic sleeve 54 which in turn is fixedly engaged by the main body of wye 26 and its distal end 56. Rigid plastic sleeve 62 is frictionally engaged by the proximal end of main branch 28. Syringe 30 frictionally engages within the inner diameter of rigid plastic sleeve 62.

Sheath 34 runs the length of secondary branch 32. It provides the lumen for optical fiber 52. Sheath 34 is sealingly engaged by stopper 58 which in turn is sealingly engaged by the proximal end of secondary branch 32. The outer diameter of sheath 34 is decreased at point 60 corresponding to the distal end of secondary branch 32. Sheath 34 terminates at aperture 50 of metal tubing 24.

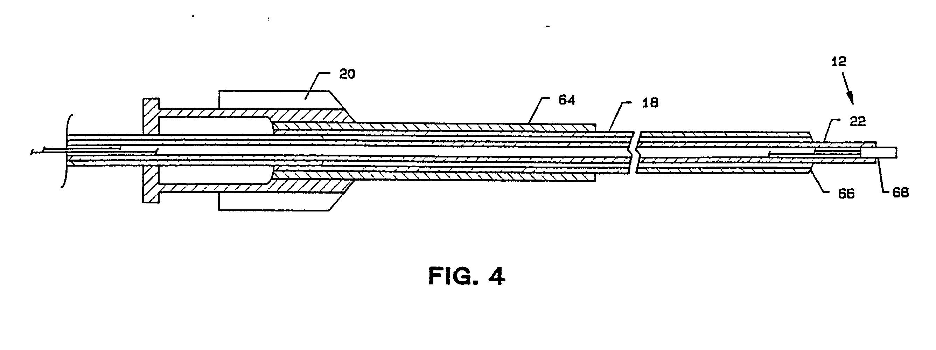

FIG. 4 is a cutaway view of the main body of catheter 10. Guiding sheath 18 runs substantially the entire length of catheter 10. Its proximal end is covered by strain relief 64 which is somewhat less flexible than guiding sheath 18, but not rigid. Guiding sheath 18 terminates at point 66 exposing inner catheter 22 which terminates at distal tip 12. Sigmoidal bend 14 and distal metal ring 16 are not shown for clarity, but may be seen in detail in FIG. 5.

FIG. 5 is a cutaway view of the distal end of catheter 10. Distal tip 12 has a metallic cylinder 68 which frictionally and adhesively engages within inner catheter 22. Metallic cylinder 68 also assists in precisely locating distal tip 12 under fluoroscopy. Optical fiber 52 is fixedly attached within the lumen of metallic cylinder 68 which also aids in energy transfer, in addition to terminating optical fiber 52. Fixation wire 42 terminates just proximal to metallic cylinder 68 when extended maximally in the distal direction. Fixation wire 42 may be advanced and retracted in the manner discussed above to assist in fixation of distal tip 12.

Sigmoidal bend 14 of guiding sheath 18 is preformed. Because guiding sheath 18 is substantially less flexible than inner catheter 22, sigmoidal bend 14 greatly aids in placement of distal tip 12 and in maintaining the desired location. Distal metal ring 16 is placed on sigmoidal bend 14. Because distal metal ring 16 is radioopaque, it is also helpful in identifying sigmoidal bend 14 during the procedure.

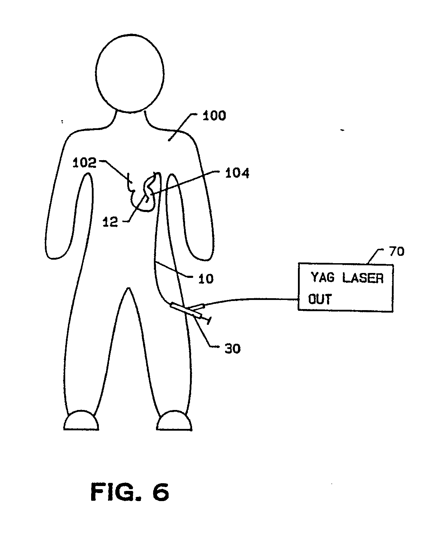

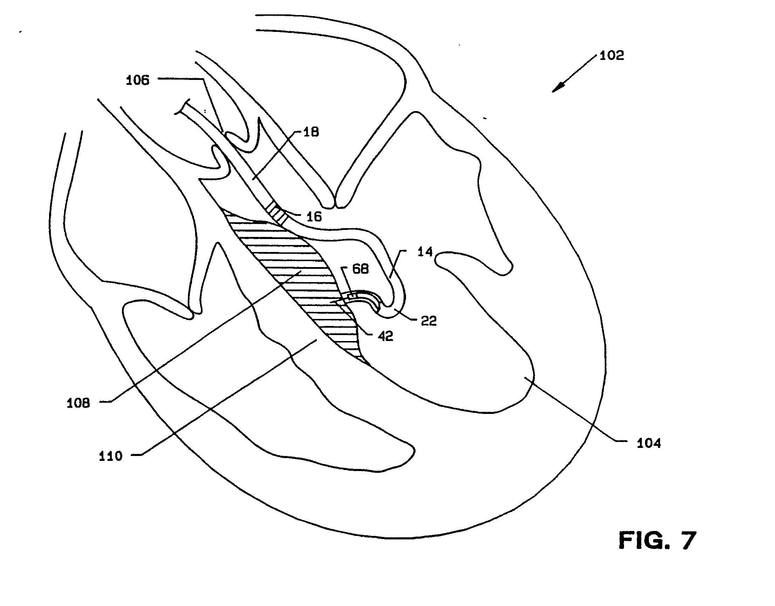

FIG. 6 is a schematic diagram of a percutaneous procedure practicing the present invention. Yag laser. 70 is preferably a Model YAG-1 manufactured and sold by Quantronix, Incorporated, although similar products are available elsewhere. Energy from YAG laser 70 is transferred via optical fiber 52 to distal tip 12 placed within left ventricle 104 of heart 102 of patient 100. In this embodiment, catheter 10 is inserted into the femoral artery and proceeds through the aorta into left ventricle 104 via the aortic valve (see also FIG. 7). During operation, the entire catheter system may be cooled by waterflow in the annular space between guiding sheath 18 and inner catheter 22.

FIG. 7 shows an enlarged cutaway view of heart 102 undergoing treatment for HCM or IHSS. As can be seen, left ventricle 104 has had its volume diminished by excessive thickness of septal wall 110 (shaded area) resulting in HCM. Furthermore, the enlargement of septal wall 110 at point 108 interferes with emptying of left ventricle 104 by occluding aortic valve 106 resulting in IHSS.

Catheter 10 has been inserted within the femoral artery as shown in FIG. 6 and has been advanced through the aorta into left ventricle 104. Notice sigmoidal bend 14 interacts with the irregular shape within left ventricle 104 to maintain the position of metallic cylinder 68 along the axis of catheter 10. Extension of fixation wire 42 prevents transverse motion. Ideally metallic cylinder 68 is positioned within 1mm of the tissue to be irradiated with the laser energy. Distal metal ring 16 aids in verification of placement using fluoroscopy. Once the exact position of metallic cylinder 68 is obtained, it is afixed by advancing thumb knob 36 as discussed above.

After correct placement of metallic cylinder 68 is verified, a short burst of laser energy is issued. Preferably the duration is approximately 15 seconds and the power is approximately 15 watts. This energy is sufficient to either cut the myocardial tissue and thereby reform it or at least produce a controlled infarct which greatly shrinks the tissue volume at the infarct area. In this fashion, the myocardium is reformed to enlarge the chamber volume and alleviate occlusion of the aortic outflow track as described by Morrow.

FIG.7A shows an enlarged cutaway view of heart 102 undergoing treatment of ventricular tachycardia using apparatus of the present invention. Heart 102 has been mapped using standard endocardial electrophysiological techniques. The mapping process identifies those areas to be irradiate.

Catheter 10 has been inserted within the femoral artery as shown in FIG. 6 and has been advanced through the aorta into left ventricle 104. Notice sigmoidal bend 14 interacts with the irregular shape within left ventricle 104 to maintain the position of metallic, cylinder 68 along the axis of catheter 10. Extension of fixation wire 42 prevents transverse motion. Ideally metallic cylinder 68 is positioned within 1mm of the tissue to be irradiated with the laser energy. Distal metal ring 16 aids in verification of placement using fluoroscopy. Once the exact position of metallic cylinder 68 is fixed by the advancing thumb knob 36 as discussed above.

After correct placement of metallic cylinder 68 is verified, a short burst of laser energy is issued.

Preferably the duration is approximately 15 second and the power is approximately 15 watts. This energy is sufficient to either ablate or photocoagulate the tissue associated with the tachycardia foci. Using standard endocardial techniques, the irradiated areas may be immediately tested to ensure that the tachycardia foci have been destroyed.


## DESCRIPTION OF AN ALTERNATIVE EMBODIMENT


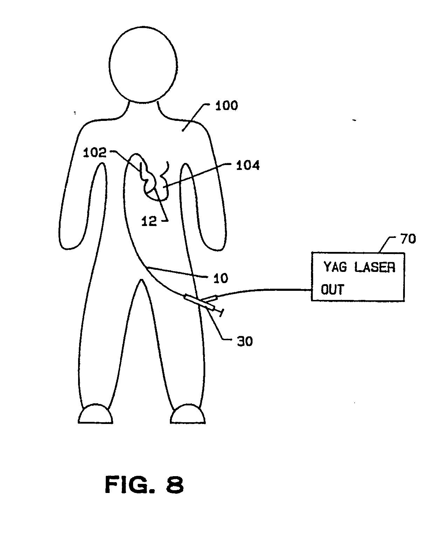

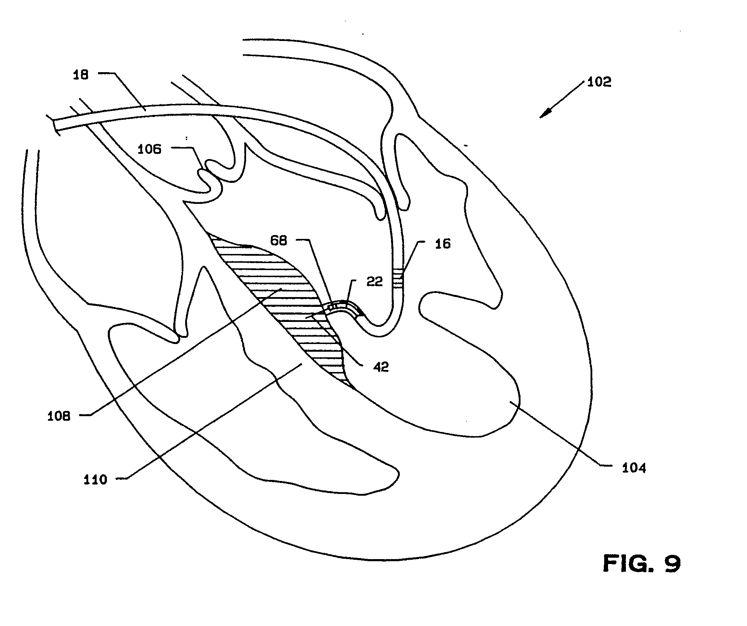

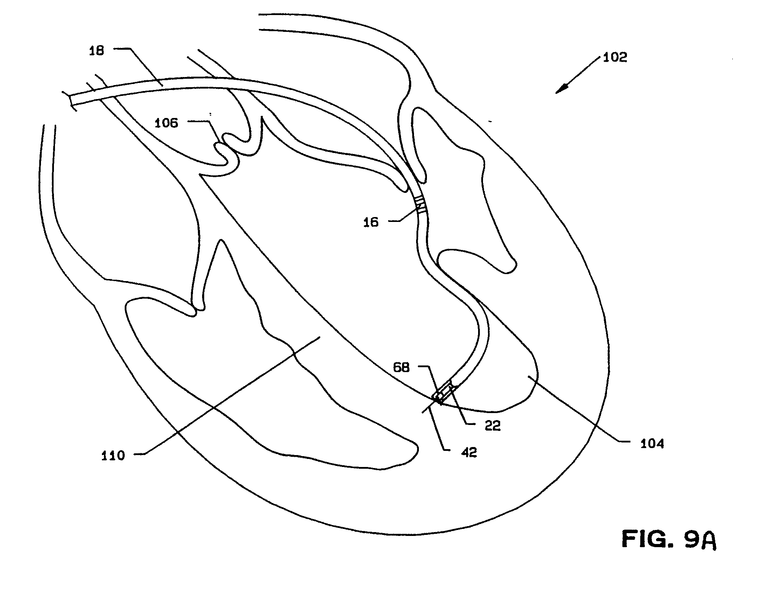

FIG. 8 shows an alternative approach to the procedure. Each of the elements is as shown in FIG. 6. The major exception is that catheter 10 is advanced to heart 102 transveneously. Insertion is preferably made into the femoral vein and is advanced to the right side of heart 102. Left ventrical 104 is entered transeptally as shown in FIG. 9.

FIG. 9 is a cutaway and enlarged view of heart 102. It differs from FIG. 7 only in that left ventrical 104 is entered transeptually as shown using procedures known in the art.

FIG. 9A is a cutaway and enlarged view of heart 102. It differs from FIG. 7A only in that left ventricle 104 is entered transeptually as shown using procedures known in the art.

The term "sigmoidal" used herein includes reference to any suitable curve, for example a curve in the general form of a 'C' or an 'S' or an ogee, or a serpentine or sinuous curve, which effects or facilitates location of the catheter with respect to the heart.

The "rigid wye" referred to herein can be regarded as comprising a first tubular portion having first and second ends and a second tubular portion which communicates with the interior of said first portion intermediate betweeen said first and second ends.

The fixation wire can be, for example, any suitable flexible elongate member capable of transmitting compressive force and preferably having an end portion suitable for being fixed (whether by piercing or otherwise) to or adjacent the treatment site.


## Claims

1. An apparatus for treating diseases of the human heart, characterised in that it comprises:
    a. a medical laser;
    b. an optical fiber (52) operatively connected to the medical laser;
    c. advancing means (30, 42) for percutaneously advancing the optical fiber to a position adjacent a human heart; and
    d. means for locating and/or maintaining the optical fiber in said position adjacent a human heart.

2. An apparatus according to Claim 1, wherein the advancing means and the maintaining means are disposed in a flexible sheath.

3. An apparatus according to Claim 1 or 2, wherein said advancing means comprises a catheter assembly 10.

4. An apparatus according to Claim 3, wherein said locating and/or maintaining means comprises a sigmoidal bend (14) in said catheter assembly.

5. An apparatus according to Claim 4, wherein said catheter assembly includes an outer sheath (18) defining a first lumen and shaped so as to provide said sigmoidal bend (14).

6. An apparatus according to Claim 5, wherein said catheter assembly includes an inner sheath or catheter (22) defining a second lumen, the inner catheter being removably located within said first lumen.

7. An apparatus according to Claim 6, wherein said optical fiber (52) is removably located within said second lumen.

8. An apparatus according to Claim 6 or 7, which includes a fixation wire (42) movably located within said second lumen.

9. An apparatus according to Claim 6, 7 or 8, wherein said inner sheath (22) has a proximal end responsively coupled to the distal end of a rigid Y-shaped housing (26) which comprises a main branch (28) and a secondary branch (32).

10. An apparatus according to Claim 9, wherein the proximal end of said main branch is responsively coupled to a piston and cylinder assembly (30).

11. An apparatus according to Claim 10, wherein the piston is responsively coupled to a proximal end of said fixation wire (42).

12. An apparatus according to Claim 8, wherein said secondary branch (32) defines a third lumen which removably contains a portion of the optical fiber.

13. An apparatus comprising:
    a. a medical laser;
    b. an optical fiber (52) responsively coupled to the medical laser;
    c. a catheter assembly (10) having an outer sheath (18) defining a first lumen and an inner sheath (22) removably located within the first lumen and defining a second lumen, the optical fiber (52) being within the second lumen and engaged to the inner sheath for advancing the optical fiber to a position adjacent a human heart; and
    d. means coupled to the catheter assembly for maintaining the optical fiber in said position adjacent a human heart.

14. A catheter assembly (10) for use in the apparatus of Claim 1 or 13, which comprises:

a first, outer flexible sheath (18);

a second, inner flexible sheath (22) removably disposed within the outer sheath and suitable to receive an optical fiber (52) and also an elongate locating and/or maintaining means (42) to locate and/or maintain a distal end of the optical fiber in or adjacent the heart;

a branched tubular housing (26) having a first tubular member (28) the distal end of which can engage the proximal end of the outer sheath (18), and a second tubular member (32) extending from the first tubular member at a position intermediate the proximal and distal ends of the first tubular member whereby the interior of the first and second tubular members are in communication with each other, the first tubular member being suitable to receive therethrough the optical fiber (52) and the second tubular member being suitable to receive therethrough the elongate means (42).

15. A catheter assembly according to Claim 14, wherein the outer sheath has a pre-formed sigmoidal bend (14) to effect or facilitate location of the catheter assembly with respect to the heart.

FIG. 1

30

36  48  42

38  46  44  40

**FIG. 2**

FIG. 3

**FIG. 4**

EP 0 416 793 A1

FIG. 5

FIG. 6

FIG. 7

EP 0 416 793 A1

FIG. 7A

EP 0 416 793 A1

FIG. 8

FIG. 9

FIG. 9A

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4 718 417 (KITTRELL et al.)<br>* Figures 16,17A; column 4, lines 40-48; column 15, lines 52,53,65-67; column 16, lines 23,45-50 *<br>— — — | 1-3 | A 61 B 17/22<br>A 61 N 1/05 |
| Y | | 4-9,11,<br>12,13 | |
| A | | 14 | |
| Y | US-A-4 103 690 (HARRIS)<br>* Figure 1; column 2, lines 41,42; column 3, lines 24-26; column 4, lines 53-57 *<br>— — — | 4,10 | |
| Y | DE-A-2 826 383 (J. EICHLER et al.)<br>* Figure 3; page 5, lines 10,11 *<br>— — — | 5 | |
| A | | 15 | |
| Y | FR-A-2 187 365 (MEDTRONIC, INC.)<br>* Figures 2,3; page 4, lines 9,10; page 6, lines 19-22 *<br>— — — | 6,7,8,13 | |
| A | | 14 | |
| Y | WO-A-8 502 101 (LASERSCOPE, INC.)<br>* Figures 2-4,7; page 10, line 26 - page 11,line 4; page 15, line 33 - page 16, line 3 *<br>— — — | 9-12 | |
| A | | 14 | TECHNICAL FIELDS SEARCHED (Int. Cl.5)<br><br>A 61 B<br>A 61 N |
| A | FR-A-2 365 351 (J. BENHAIM)<br>* Figure 2; page 3, lines 31-39 *<br>— — — | 2,3 | |
| A | DE-A-3 718 139 (GESELLSCHAFT FÜR STRAHLEN- UND UMWELTFORSCHUNG mbH)<br>* Figures 1,2; column 2, lines 40-55 *<br>— — — | 2 | |
| A | EP-A-0 048 410 (OLYMPUS OPTICAL CO., LTD)<br>* Figure 1; page 4, lines 31-36 *<br>— — — | 7,8 | |
| | — / — | | |

The present search report has been drawn up for all claims

| Place of search | Date of completion of search | Examiner |
|---|---|---|
| The Hague | 08 December 90 | SEDY, R. |

European
Patent Office

**EUROPEAN SEARCH
REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | DE-A-3 527 451   (OLYMPUS OPTICAL CO., LTD)<br>* Figure 4; page 10, lines 16-27; page 11, lines 26-33 *<br>– – – – – | 8 | |

TECHNICAL FIELDS
SEARCHED (Int. Cl.5)

The present search report has been drawn up for all claims

| Place of search | Date of completion of search | Examiner |
|---|---|---|
| The Hague | 08 December 90 | SEDY, R. |

CATEGORY OF CITED DOCUMENTS

X : particularly relevant if taken alone
Y : particularly relevant if combined with another
document of the same catagory
A : technological background
O : non-written disclosure
P : intermediate document
T : theory or principle underlying the invention

E : earlier patent document, but published on, or after
the filing date
D : document cited in the application
L : document cited for other reasons

 

& : member of the same patent family, corresponding
document